# EUROPÄISCHE PATENTANMELDUNG

(11) **EP 3 354 297 A1**
(43) Veröffentlichungstag der Anmeldung: **01.08.2018**
(21) Anmeldenummer: 18153377.9
(22) Anmeldetag: 25.01.2018
(51) Int. Cl.: A61M 1/16, A61M 5/14

(54) **VORRICHTUNG ZUR HALTERUNG EINER MEDIZINTECHNISCHEN KOMPONENTE**

(30) Priorität: 27.01.2017 DE 102017000893
(71) Anmelder: seca ag, 4153 Reinach BL 1 (CH)
(72) Erfinder: LEUNER, Rüdiger, 21107 Hamburg (DE); WIETZKE, Timo, 20259 Hamburg (DE)
(74) Vertreter: Klickow & Wetzel PartGmbB

(57) **Zusammenfassung**

Die Vorrichtung dient zur Halterung einer medizintechnischen Komponente und weist einen mit einem Träger verbindbaren Basisbereich sowie zwei sich ausgehend vom Basisbereich erstreckende Seitenbereiche auf. Der Basisbereich bildet gemeinsam mit den Seitenbereichen eine klammerartige Struktur aus. Die klammerartige Struktur ist hinsichtlich ihrer Formgebung an eine Außengeometrie der zu halternden Komponente angepasst.

## Beschreibung

Die Erfindung betrifft eine Vorrichtung zur Halterung einer medizintechnischen Komponente, die einen mit einem Träger verbindbaren Basisbereich sowie zwei sich ausgehend vom Träger erstreckende Seitenbereiche aufweist.

Derartige Vorrichtungen sind bereits in unterschiedlichen Ausführungsformen zur Halterung unterschiedlicher medizintechnischer Komponenten bekannt. Die konkrete Konstruktion und Dimensionierung hängt hierbei von den Anwendungsanforderungen ab.

Typischerweise werden an derartige Vorrichtungen die Anforderungen gestellt, dass eine zuverlässige und technisch robuste Fixierung der medizintechnischen Komponente erreicht werden soll, dass eine einfache Bedienbarkeit und eine einfache Reinhaltbarkeit gewährleistet werden müssen und dass schließlich auch eine Montage und Demontage in einfacher Weise erfolgen kann. Die Kombination derartiger Anforderungen lässt sich mit den bekannten Vorrichtungen noch nicht in vollständig zufriedenstellender Weise lösen.

Aufgabe der vorliegenden Erfindung ist daher, eine Vorrichtung der einleitend genannten Art derart zu konstruieren, dass verbesserte Gebrauchseigenschaften bereitgestellt werden.

Diese Aufgabe wird erfindungsgemäß dadurch gelöst, dass der Basisbereich gemeinsam mit den Seitenbereichen eine klammerartige Struktur ausbildet, die hinsichtlich ihrer Formgebung an eine Außengeometrie der zu halternden Komponente angepasst ist.

Die erfindungsgemäße Konstruktion ermöglicht es, die medizintechnische Komponente in einfacher Weise an und durch die Vorrichtung zu fixieren und ebenfalls in einfacher Weise die Komponente wieder von der Vorrichtung zu trennen, um eine vorgesehene Handhabung durchzuführen.

Gemäß einer bevorzugten Ausführungsform ist daran gedacht, die Vorrichtung zur Halterung einer sogenannten Netzwerkbox zu verwenden, die ein medizintechnisches Gerät mit einer Einrichtung zur Messwerterfassung und/oder Messwertverarbeitung verbinden kann. Gemäß einer weiteren bevorzugten Ausführungsform ist die Vorrichtung zur Verwendung im Bereich einer Waage oder eines Body composition analyzers (BCA) vorgesehen.

Eine preiswerte Fertigung und eine einfache Handhabung werden dadurch unterstützt, dass der Basisbereich und die Seitenbereiche einteilig die klammerartige Struktur ausbilden.

Eine bevorzugte Materialauswahl besteht darin, dass der Basisbereich und die Seitenbereiche aus Kunststoff ausgebildet sind.

Eine einfache Montierbarkeit und Demontierbarkeit wird dadurch unterstützt, dass der Basisbereich mit dem Träger verschraubbar ist.

Gemäß einer bevorzugten Ausführungsform ist daran gedacht, dass der Basisbereich gemeinsam mit den Seitenbereichen mindestens bereichsweise einen Aufnahmeraum ovalartig begrenzt.

Eine hohe Benutzungssicherheit wird dadurch unterstützt, dass mindestens einer der Seitenbereiche zur Abdeckung mindestens eines Bedienelementes der Komponente ausgebildet ist.

Gemäß einer weiteren Ausführungsform ist vorgesehen, dass eine Elastizität der klammerartigen Struktur zur Bereitstellung einer mechanischen Überlastsicherung dimensioniert ist.

Die Erfindung wird im Folgenden anhand einiger exemplarischer Darstellungen verdeutlicht. Es zeigen:
- Fig. 1: eine perspektivische Darstellung einer Vorrichtung zur Halterung einer medizintechnischen Komponente, wobei die Vorrichtung unter Verwendung eines Nutensteins im Bereich einer Halterungsnut eines Trägers fixierbar ist,
- Fig. 2: eine perspektivische Darstellung der Vorrichtung gemäß Figur 1 bei einer Blickrichtung schräg von hinten,
- Fig. 3: eine Darstellung ähnlich zu Figur 1 bei der Realisierung einer Fixierung der Vorrichtung im Bereich eines Trägers unter Verwendung eines Zwischenelementes,
- Fig. 4: ein Beispiel für die Verwendung der Vorrichtung im Bereich einer Waage und
- Fig. 5: eine weitere Anordnung der Vorrichtung im Bereich einer Waage zur Veranschaulichung der Realisierung einer mechanischen Überlastsicherung.

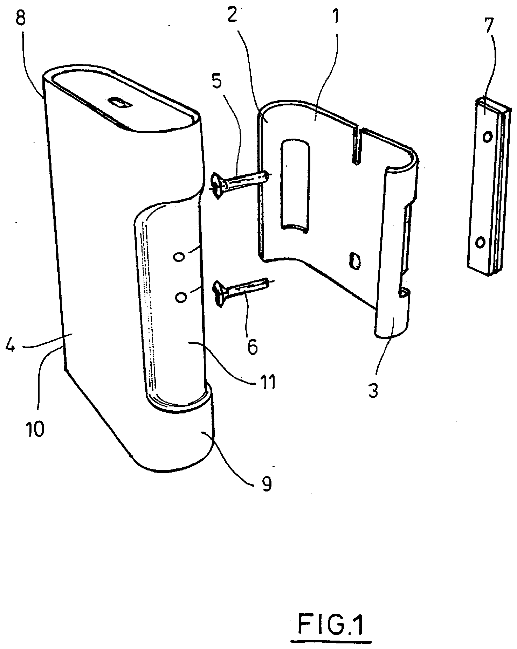

Gemäß der Ausführungsform in Figur 1 ist die Vorrichtung klammerartig ausgebildet und besitzt einen Basisbereich (1) sowie zwei sich ausgehend vom Basisbereich (1) erstreckende Seitenbereiche (2, 3) auf. Der Basisbereich (1) bildet gemeinsam mit den Seitenbereichen (2, 3) eine klammerartige Struktur aus. Eine Formgebung dieser klammerartigen Struktur ist an eine Außengeometrie einer zu halternden medizintechnischen Komponente (4) angepasst.

Unter Verwendung von Schrauben (5, 6) kann der Basisbereich (1) mit einem Nutenstein (7) verbunden werden.

Beim dargestellten Ausführungsbeispiel ist die Komponente (4) als eine Box ausgebildet, die zu Kommunikationszwecken verwendbar ist. Im Bereich von zwei Flanken (8, 9) weist die Komponente (4) jeweils muldenartige Vertiefungen (10, 11) auf. Hinsichtlich ihrer geometrischen Gestaltung sind die Seitenbereiche (2, 3) an die Geometrie der Vertiefungen (10, 11) angepasst.

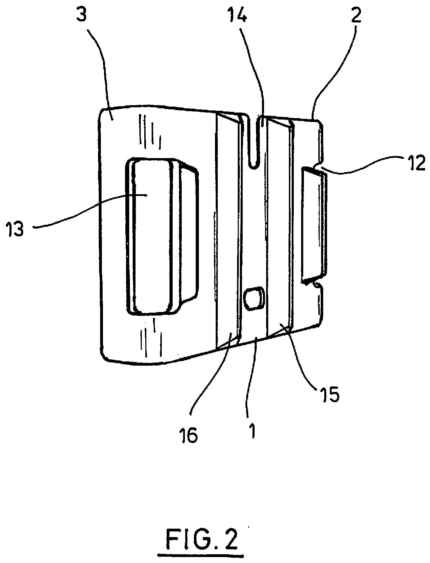

Gemäß der Darstellung in Figur 2 weisen die Seitenbereiche (2, 3) Ausnehmungen (12, 13) auf. Diese dienen zum einen zur Materialeinsparung, darüber hinaus werden den Seitenbereichen (2, 3) zusätzliche federnde Eigenschaften verliehen. Der Basisbereich (1) besitzt im Bereich einer Rückseite (14) zwei im Wesentlichen parallel zueinander angeordnete Stege (15, 16), die eine Montage der Vorrichtung erleichtern und eine Ausrichtung unterstützt.

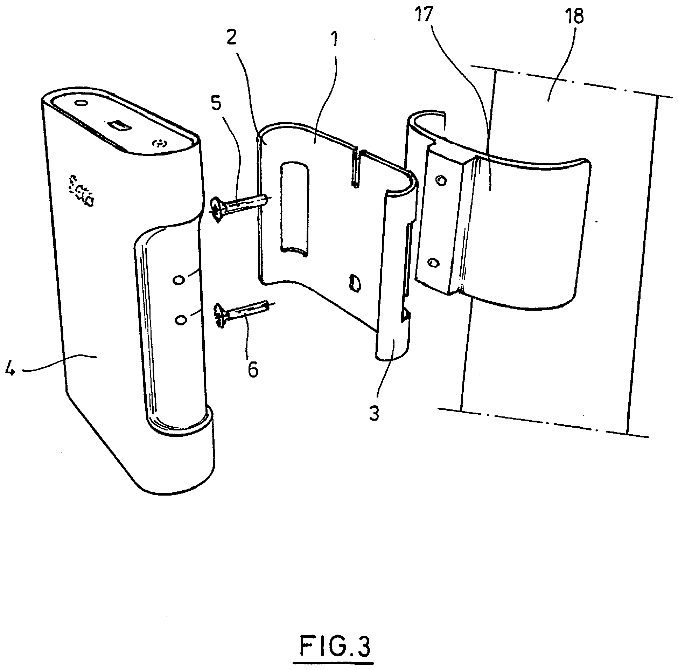

Gemäß der Ausführungsform in Figur 3 ist es möglich, die Vorrichtung unter Verwendung eines Zwischenelementes (17) im Bereich eines Trägers (18) anzuordnen. Das Zwischenelement (17) kann hierbei federnd auf den Träger (18) aufgeschoben werden. Eine Verbindung der Vorrichtung mit dem Zwischenelement kann beispielsweise unter Verwendung der Schrauben (5, 6) erfolgen.

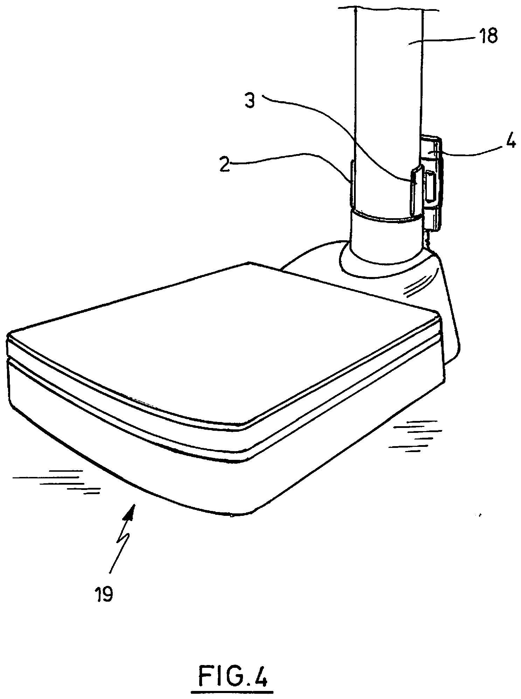

Gemäß dem Ausführungsbeispiel in Figur 4 ist die Vorrichtung gemeinsam mit einer gehalterten Komponente (4) im Bereich einer Waage (19) angeordnet. Der Träger (18) ist hierbei als Teil der Waage (19) realisiert.

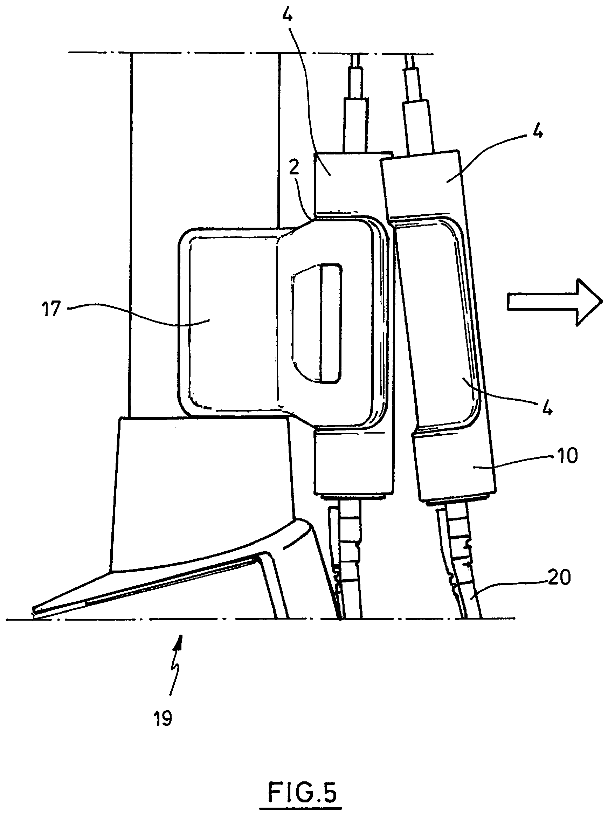

Gemäß dem Ausführungsbeispiel in Figur 5 erfolgt ebenfalls eine Anordnung der Vorrichtung und der Komponente (4) im Bereich einer Waage (19). Durch die federnde Halterung der Komponente (4) unter Verwendung der Seitenbereiche (2, 3) ist es möglich, eine Überlastsicherung zu realisieren. Bei Aufbringung einer vorgebbaren Kraft, beispielsweise auf ein Anschlusskabel (20), wird eine Trennung der Komponente (4) von der klammerartigen Vorrichtung realisiert. Hierdurch wird insbesondere eine Beschädigung einer das Anschlusskabel (20) mit der Komponente (4) verbindenden Kupplung vermieden.

## Patentansprüche

1. Vorrichtung zur Halterung einer medizintechnischen Komponente, die einen mit einem Träger verbindbaren Basisbereich sowie zwei sich ausgehend vom Basisbereich erstreckende Seitenbereiche aufweist, **dadurch gekennzeichnet, dass** der Basisbereich (1) gemeinsam mit den Seitenbereichen (2, 3) eine klammerartige Struktur ausbildet, die hinsichtlich ihrer Formgebung an eine Außengeometrie der zu halternden Komponente (4) angepasst ist.

2. Vorrichtung nach Anspruch 1, **dadurch gekennzeichnet, dass** der Basisbereich (1) und die Seitenbereich (2, 3) einteilig die klammerartige Struktur ausbilden.

3. Vorrichtung nach einem der Ansprüche 1 oder 2, **dadurch gekennzeichnet, dass** der Basisbereich (1) und die Seitenbereiche (2, 3) aus Kunststoff ausgebildet sind.

4. Vorrichtung nach einem der Ansprüche 1 bis 3, **dadurch gekennzeichnet, dass** der Basisbereich (1) mit dem Träger (18) verschraubbar ist.

5. Vorrichtung nach einem der Ansprüche 1 bis 4, **dadurch gekennzeichnet, dass** der Basisbereich (1) gemeinsam mit den Seitenbereichen (2, 3) mindestens bereichsweise einen Aufnahmeraum ovalartig begrenzt.

6. Vorrichtung nach einem der Ansprüche 1 bis 5, **dadurch gekennzeichnet, dass** mindestens einer der Seitenbereiche (2, 3) zur Abdeckung mindestens eines Bedienelementes der Komponente (4) ausgebildet ist.

7. Vorrichtung nach einem der Ansprüche 1 bis 6, **dadurch gekennzeichnet, dass** eine Elastizität der klammerartigen Struktur zur Bereitstellung einer mechanischen Überlastsicherung dimensioniert ist.
